Europäisches Patentamt

European Patent Office

Office européen des brevets

(19)

(11) Numéro de publication : **0 412 025 B1**

## (12) FASCICULE DE BREVET EUROPEEN

(45) Date de publication du fascicule du brevet :
**24.06.92 Bulletin 92/26**

(51) Int. Cl.⁵ : **B65D 81/32,** B01J 13/00

(21) Numéro de dépôt : **90420344.5**

(22) Date de dépôt : **18.07.90**

(54) **Procédé d'encapsulation et libération d'une matière active et micro-capsules obtenues.**

(30) Priorité : **02.08.89 FR 8910631**

(43) Date de publication de la demande :
**06.02.91 Bulletin 91/06**

(45) Mention de la délivrance du brevet :
**24.06.92 Bulletin 92/26**

(84) Etats contractants désignés :
**AT BE CH DE DK ES FR GB GR IT LI LU NL SE**

(56) Documents cités :
**US-A- 4 806 371**

(73) Titulaire : **SOCIETE ANONYME DES PAPETERIES DE CLAIREFONTAINE
F-88480 Etival Clairefontaine (FR)**

(72) Inventeur : **Pouyet, Bernard
501 avenue du 8 mai
F-69300 Caluire (FR)**

(74) Mandataire : **Guerre, Dominique et al
Cabinet Germain et Maureau 20 Boulevard
Eugène Deruelle BP 3011
F-69392 Lyon Cédex 03 (FR)**

## Description

L'invention concerne, d'une part un procédé d'encapsulation d'une matière active, et de libération de cette dernière, et d'autre part des micro-capsules obtenues selon ce procédé, et permettant sa mise en oeuvre.

Par "matière active", au sens de la présente invention, on entend tout produit ayant une application pratique ou industrielle, ou utilité, c'est-à-dire ayant une fonction ou apportant un résultat dans tout procédé, méthode, processus, de nature physique, chimique, biologique, biochimique, pharmaceutique, thérapeutique, etc... La nature et la structure physiques ou chimiques de cette matière active peuvent être diverses ; il peut s'agir d'un composé minéral, organique, biochimique, etc... Toutefois, cette matière active doit être compatible avec le procédé d'encapsulation puis libération selon l'invention, explicité ci-après, ce que des essais de routine pour l'homme de métier peuvent mettre en évidence. A titre de matière active, on peut par exemple retenir les produits suivants :

– encres, pigments, colorants
– colles et adhésifs
– produits chimiques, jouant un rôle par exemple dans une synthèse, un traitement, etc...
– principes actifs pharmaceutiques, thérapeutiques, et de diagnostic, etc...

Par "micro-capsules", on entend des vésicules fermées, par exemple sphériques, creuses à l'intérieur, ayant différentes dimensions s'échelonnant de quelques $\mu$m à quelques mm. Ces micro-capsules sont obtenues selon les techniques traditionnelles de micro-encapsulation bien décrites dans la littérature, et par exemple dans :

– le document FR-A- 2 548 046
– le document BE-A- 796 746
– les documents US-C- 3 790 497, 3 577 515, 3 173 878
– Journal of micro-encapsulation Vol 1, N° 1, Microcapsules processing and technology - Asaji Kondo

L'enveloppe de ces capsules, en général imperméable par rapport à leur contenu, est obtenue par polymérisation d'un monomère ou plusieurs monomères différents, à l'interface en milieu dispersé de deux phases distinctes, dont l'une est constituée par la matière active à encapsuler, et éventuellement par un milieu interne de dissolution ou suspension de cette dernière, et ce en général sous forte agitation. Les voies chimiques de polymérisation peuvent être traditionnelles, par exemple radicalaires, ioniques, polyaddition, polycondensation, etc... L'enveloppe polymérique obtenue peut être ou non élastique. Toutes sortes de polymères peuvent être retenus, par exemple l'EMA, à savoir copolymère éthylène-anhydridre maléique, l'éthyl-cellulose, un polyamide, une polyureé, etc...

Par "milieu interne", on entend une substance liquide ou fluide, contenue par les micro-capsules. Il peut s'agir d'une substance distincte de la matière active, cette dernière pouvant être alors en solution, suspension, dispersion, ou émulsion dans ladite substance. Mais la matière active peut former ou constituer à elle-seule le milieu interne, si elle-même est liquide ou fluide.

Ces micro-capsules, renfermant la matière active, peuvent être utilisées directement en tant que telles, notamment en mélange à d'autres produits. Elles peuvent être aussi mises en oeuvre ou appliquées sous la forme de différents complexes ; de tels complexes comprennent par exemple un substrat sous forme de feuille, et un revêtement comportant les micro-capsules, sous forme liée au substrat ; il s'agit par exemple de feuilles de reproduction sur lesquelles sont couchées des micro-capsules incorporant un pigment ou colorant. D'autres complexes comprennent ces micro-capsules en solution dans un liquide ou fluide, lequel préserve l'intégrité de l'enveloppe de ces micro-capsules ; il s'agit par exemple de pâtes d'impression mélangées à des micro-capsules incorporant une encre.

Différents processus ou agents extérieurs peuvent être utilisés pour rompre l'enveloppe des micro-capsules. Et il convient de les examiner, pour en situer les inconvénients respectifs.

Le premier moyen consiste à faire éclater les micro-capsules par voie mécanique, notamment par pression appliquée sur l'enveloppe, ou par abrasion sur une brosse.

L'éclatement par voie mécanique des micro-capsules requiert l'application d'une pression régulière, et homogène, dans un plan par exemple. A cet égard, d'une part ceci requiert l'usage d'un équipement de compression de précision, et un réglage fin, et d'autre part, l'irrégularité d'un éventuel support, à l'échelle des micro-capsules en creux et en bosses, contrarie l'application d'une pression homogène dans le plan de compression. Tout ceci aboutit donc à un éclatement des micro-capsules, erratique quant à sa localisation, ce qui exclut l'application de ce moyen d'éclatement aux procédés requérant la libération de la matière active à un endroit précis.

Cet éclatement par voie mécanique n'est en pratique opérationnel qu'avec des micro-capsules présentant une certaine taille, par exemple supérieures à 100 $\mu$m. Des micro-capsules ayant des dimensions aussi importantes peuvent être incompatibles avec le procédé faisant usage de la libération de la matière active. Il apparaît dans certaines applications souhaitable d'utiliser des micro-capsules aussi fines que possible.

Pour terminer, l'épaisseur de l'enveloppe des micro-capsules peut varier de manière significative d'un individu à un autre. Or, toutes les capsules sont soumises unitairement par l'extérieur à la même compression. Il en résulte que certaines capsules éclateront, d'autres pas, et ceci de manière toujours erratique et incontrôlée, ce qui nuit à la maîtrise et à la précision du processus intégrant la libération de la matière active.

Le second moyen d'éclatement consiste à incorporer une substance porofore dans le milieu interne des micro-capsules, l'enveloppe polymérique étant transparente à un rayonnement ultra-violet. En soumettant les micro-capsules à un tel rayonnement, la substance porofore dégage un gaz à l'intérieur des micro-capsules et les fait éclater.

L'usage d'un rayonnement ultra-violet peut être incompatible avec la matière active, au sens où il endommage cette dernière ou la dégrade. Compte-tenu de la nocivité des rayons ultra-violets, l'application d'une lumière ultra-violette requiert des équipements protégés, donc coûteux et d'une mise en oeuvre délicate. Pour terminer, les substances porofores traditionnelles présentent de sérieuses difficultés de solubilisation dans un milieu aqueux interne aux micro-capsules, et elles doivent être stockées à basse température.

Le troisième moyen d'éclatement consiste à utiliser un milieu interne vaporisable, par exemple à faible tension de vapeur, et à soumettre les micro-capsules à l'action de la chaleur. L'action de la chaleur peut être incompatible avec la matière active, au sens où celle-ci est affectée ou dégradée par la chaleur.

Il faut noter, de manière commune au premier et au deuxième moyens d'éclatement, que ces derniers sont à action lente, et par conséquent incompatibles avec des procédés mécaniques ou automatisés requérant une quasi-instantanéité de l'éclatement.

Le quatrième et dernier moyen consiste à utiliser un solvant de dissolution de l'enveloppe polymérique des micro-capsules. Là encore, il s'agit d'un moyen à action lente, nécessitant de travailler en milieu liquide, ce qui peut être une contrainte importante dans certains procédés.

La présente invention a pour objet de remédier aux différents inconvénients explicités précédemment.

La présente invention a pour objet des micro-capsules pouvant être éclatées de manière exhaustive, quelles que soient les irrégularités ou imperfections tant intérieures qu'extérieures, ou de leurs différents composants.

Selon l'invention, au moins l'un des composants des micro-capsules, à savoir l'enveloppe, éventuellement le milieu interne, et la matière active, est susceptible d'absorber et de s'échauffer sous l'action d'un rayonnement électro-magnétique hyper-fréquence, dit rayonnement micro-onde. Cet échauffement conduit à la rupture des mêmes micro-capsules, avec libération vers l'extérieur de la matière active. Par conséquent selon l'invention, le rayonnement micro-onde est utilisé comme moyen d'éclatement des micro-capsules.

Un tel procédé apporte en outre les avantages déterminants suivants.

La nature endogène, c'est-à-dire de l'intérieur vers l'extérieur, de l'action des micro-ondes, provoquant l'éclatement des micro-capsules, à savoir la chaleur générée dans ces dernières par l'action des micro-ondes, affranchit le processus d'éclatement de toutes les contraintes traditionnelles et extérieures, liées à la taille des capsules, leur régularité, et leurs dimensions unitaires, mais aussi liées à l'usage de moyens externes d'éclatement.

L'éclatement sous l'action des micro-ondes se révèle être très rapide, quasi-instantané, et est alors compatible avec une automatisation ou mécanisation.

L'action des micro-ondes évite de recourir à tout solvant, ou autres matières organiques liquides, polluantes, par exemple pour dissoudre l'enveloppe des micro-capsules. Il s'agit donc d'un procédé pouvant être mis en oeuvre "à sec", ce qui aussi est très intéressant dans certaines applications.

L'action des micro-ondes est sélective, et n'altère pas les propriétés ou caractéristiques des composants des micro-capsules, autres que ceux absorbant le rayonnement hyper-fréquence. Ceci permet si besoin est de préserver les propriétés de la matière active.

Avec l'invention, on peut régler la puissance des micro-ondes de façon à moduler l'éclatement des micro-capsules.

Au total, l'invention offre un procédé d'éclatement, facile à mettre en oeuvre, et relativement peu coûteux quant aux moyens techniques qui doivent lui être associés.

Pour que les micro-capsules selon l'invention absorbent le rayonnement micro-onde, et s'échauffent sous l'effet de ce dernier, différentes solutions peuvent être retenues :

– ce peut être tout d'abord l'enveloppe des micro-capsules qui absorbe ce rayonnement, par exemple si celle-ci est faite en un matériau chimique polaire

– ce peut être ensuite le milieu interne des micro-capsules qui absorbe ce même rayonnement

– pour terminer, ce peut être aussi la matière active qui absorbe le rayonnement micro-onde, par exemple s'il s'agit d'un corps chimique comportant des groupements fonctionnels polaires, par exemple alcools, amines, et acides ; mais la matière active peut aussi absorber le rayonnement micro-onde, s'il s'agit d'un matériau métallique

Par "rayonnement micro-onde", on entend un rayonnement électro-magnétique dont les longueurs d'onde sont inférieures à 1 cm, ou dont les fréquences sont supérieures à 1 GHz.

La technologie propre à la génération et à l'utilisation de ces rayonnements est par exemple décrite dans : H.F. Mark, D.F. Gthmer et al, Kirk Gthmer Encyclopedia of Chemical Technology, Edition 3, n° 15, 1989, Wiley and Sons, New York, U.S.A., page 505 à page 521.

Par "matériau polaire", ou "groupement fonctionnel polaire", on entend tout agencement d'atomes polarisé électriquement en raison de la nature chimique desdits atomes, et se comportant comme un dipole lorsqu'il est placé entre deux électrodes de polarité opposées. On sait qu'en présence de micro-ondes, de tels agencements sont mis en mouvement, notamment en rotation, ce qui conduit à l'échauffement du matériau correspondant.

Au plan expérimental, on a tout d'abord recherché et mis au point différentes techniques de micro-encapsulation, conduisant à des micro-capsules perméables a un rayonnement micro-ondes.

Trois techniques retenues sont répertoriées ci-après.

## 1) PROCEDE A L'EMA

### PRINCIPE

Cette méthode utilise un polymère particulier : l'EMA, copolymère éthylène-anhydridre maléique qui dans une certaine zone de pH interréagit avec une résine mélamine-formol pour former la paroi des micro-capsules.

### MANIPULATION

Dans un réacteur en verre, on agite vigoureusement en chauffant une solution aqueuse (480g) contenant de la soude à 50 % (11,62g), ainsi que le copolymère EMA (15g). Lorsque le polymère est totalement solubilisé (solution translucide), la solution est refroidie à 30° C. Le pH doit alors être compris entre 5,8 et 6,2. On ajoute alors successivement, sous agitation :

– de la n-butylamine (8,3 g) le pH est alors compris entre 10,5 et 11

– une résine mélamine formol (poids à 45 % : 51,1 g)

– de l'acide acétique jusqu'à l'obtention d'un pH inférieur à 4,5.

L'agitation est stoppée. La solution se présente sous la forme d'un lait. La solution à encapsuler (280 g) est ajoutée à la solution précédente. Celle-ci comporte le produit à encapsuler solubilisé dans un solvant adéquat. Après décantation et disparition des mousses, l'opération d'encapsulation peut avoir lieu. La turbine microdisperseuse est mise au maximum de sa puissance (12000 t/mn) pendant 10 mn.

La solidification des parois des capsules se fait ensuite par chauffage de l'émulsion à 95° C pendant une heure.

La neutralisation de l'excès d'acidité de l'émulsion se fait après refroidissement à 30° C avec de l'ammoniac dilué jusqu'à pH 7.

### RESULTATS

Cette technique d'encapsulation est simple et rapide. Elle permet l'obtention de capsules régulières et assez solides. Ces capsules présentent un diamètre de 20 à 50 microns, et la distribution des tailles à l'intérieur d'une même solution est assez homogène

## 2° PROCEDE PAR POLYMERISATION INTERFACIALE

### PRINCIPE

La micro-encapsulation par polymérisation interfaciale se fait en général entre une amine RNH2 et un acide R'COOH pour donner une amide RCONHR'. Ce processus est appelé polymérisation interfaciale simple.

Il est possible d'employer un triacide ou une triamine afin de consolider la liaison ainsi formée, en créant un réseau trifonctionnel. Il s'agit de la polymérisation interfaciale complexe.

Dans le but de l'application des micro-ondes selon l'invention, on retient la polymérisation interfaciale complexe.

MANIPULATION

On prépare les trois solutions suivantes.

Solution 1 :

– 115 ml d'huile minérale (type vaseline)
– 50 ml de solvant CC14

Solution 2 :

– 0,075 g d'éthylène diamine
– 0,075 g de diéthylène triamine
– 0,5 g de carbonate de sodium hydraté
– 6,5 ml d'eau (+ agent colorant)

Solution 3 :

– 0,021 g de chlorure de sébacoyle
– 0,04 g de trichlorure de trimésoyle
– 12,5 g de CC14
– 12,5 g de pentane

On verse la solution 2 dans la solution 1 sous agitation rapide pendant 1 minute, à température ambiante. On ajoute alors la solution 3 sous agitation de type ancre pendant 1 heure, à température ambiante.

Afin d'obtenir des micro-capsules non agglomérées, il est important de maintenir constants les rapports éthylène diamine/chlorure de sébacoyle = 3,5 et diéthylène triamine/trichlorure de trimésoyle = 2. Toutefois, on peut faire varier les quantités dans des rapports de 1 à 10.

On obtient alors des murs de polymères plus ou moins importants, et les micro-capsules présentent naturellement des aspects différents à l'observation au microscope optique.

CONCLUSION

Ce mode d'encapsulation donne des résultats très satisfaisants pour l'encapsulation de substances aqueuses selon l'invention. On obtient en effet des capsules non agglomérées qui présentent une paroi hermétique mais transparente. Il semble que l'on puisse moduler le diamètre des capsules, aussi bien que l'épaisseur des parois, en fonction de l'application.

## 3) PROCEDE PAR POLYCONDENSATION INTERFACIALE

PRINCIPE

Selon le processus chimique :

$$\begin{array}{ccc} \text{diamine ou} & \text{diisocyanate ou} & = \text{Polyurée} \\ \text{polyamine} & + & \text{polyisocyanate} \end{array}$$

On réalise l'hydrolyse d'un isocyanate pour former une amine qui à son tour réagit avec le même isocyanate pour former une polyurée.

MANIPULATION

On prépare la phase dispersante par dissolution et homogénéisation de :
– 400 cm³ de xylène
– 10 g de 22-éthyl-monoanhydrosorbitol-isocyanate trioléate (émulsifiant oléophile)
– 12 g d'un polyméthylène-polyphényl-isocyanate commercialisé par la société Bayer sous l'appellation de Desmodur R 44 V 20, et dont la fonctionnalité moyenne en groupe NCO par mole est de 2,7.

5

On émulsionne 70 cm3 d'eau distillée dans le mélange préparé ci-avant, au moyen d'une turbine tournant à 4500 t/mn. Au bout de 40 secondes on obtient une émulsion stable. La turbine est arrêtée et l'essai se poursuit sous agitation de type ancre.

On laisse poursuivre la réaction pendant 3 heures à température ambiante. On laisse ensuite décanter. Les micro-capsules sont filtrées puis lavées au cyclohexane, puis par un mélange 50/50 en poids d'eau et de 22 diéthyl-monoanhydrosorbitol monolaurate, puis par de l'eau pure.

Des variantes sont apportées à ce mode opératoire, avec la possibilité de stabiliser l'émulsion avec de la carboxyméthylcellulose ou du silicate de soude (à un pH donné).

CONCLUSION

Cette méthode permet d'obtenir des micro-capsules de type eau dans huile, de très faible diamètre (5 à 10 microns), ayant une paroi d'épaisseur convenable, c'est-à-dire permettant une bonne imperméabilisation des micro-capsules.

On a ensuite recherché un composé photo-polymérisable adéquat, selon la procédure suivante :

ESSAIS

Les matières premières testées sont les suivantes :
– Monomère : acrylamide (soluble dans l'eau)
– Photoinitiateur : 1 phényl 1-2 propanédione 2- (0-éthoxycarbonyl)oxime, benzophénones, thioxanthones combinées à une amine tertiaire
– Relais : sel de sodium de l'acide p-toluène sulfinique à 99 %.

CONCLUSIONS :

Les benzophénones et thioxanthones apparaissent à priori adaptées aux applications selon l'invention.

On a ensuite procédé aux expériences suivantes de micro-encapsulation, avec éclatement sous rayonnement micro-ondes.

Expérience n° 1

– Réalisation par polycondensation interfaciale de micro-capsules à paroi en polyurée, de diamètre compris entre 5 et 10 microns, à coeur aqueux selon le procédé RHONE POULENC (brevet français 2 548 046)
– Ces micro-capsules sont en solution dans une phase organique (xylène et tensio-actif) et déposées sur une lamelle transparente.
– Cette lamelle est déposée au centre d'une cavité à mode TE 10, et irradiée au moyen d'un générateur magnetron de 2,45 Giga Hertz pendant 10 secondes.
– Après observation au microscope optique, on constate que ces capsules éclatent par rupture de la paroi en libérant leur contenu.

Expérience n° 2

– Réalisation par polycondensation interfaciale (paroi en polyamide) de micro-capsules de 40 microns selon le brevet US 3 577 515, exemple 5.
– Ces micro-capsules sont en solution dans une phase organique (huile minérale/CC14), et déposées au fond d'un tube à essais.
– Ce tube est déposé au fond d'une cavité du même type que celle décrite précédemment, pendant 5 secondes.
– Après observation au microscope optique, on constate de même l'éclatement de l'ensemble des micro-capsules.

**Revendications**

1. Micro-capsule comportant une enveloppe étanche susceptible de se rompre sous l'action d'un agent extérieur, une matière active, et éventuellement un milieu interne contenus par ladite enveloppe, <u>caractérisée</u>

en ce qu'au moins l'un des composants de la micro-capsule, à savoir l'enveloppe, la matière active, et éventuellement le milieu interne, est susceptible d'absorber et de s'échauffer sous l'effet d'un rayonnement électro-magnétique hyper-fréquence, dit rayonnement micro-onde, et de conduire par son échauffement à la rupture de la même micro-capsule, avec libération vers l'extérieur de la matière active.

2. Micro-capsule selon la revendication 1, caractérisée en ce que le composant absorbant le rayonnement micro-onde est l'enveloppe de ladite micro-capsule.

3. Micro-capsule selon la revendication 2, caractérisée en ce que l'enveloppe est faite en un matériau chimique polaire.

4. Micro-capsule selon la revendication 1, caractérisée en ce que le composant absorbant le rayonnement micro-onde est la matière active.

5. Micro-capsule selon la revendication 1, contenant un milieu interne liquide dans lequel la matière active est dissoute ou en suspension, caractérisée en ce que le composant absorbant le rayonnement micro-onde est le milieu interne de la micro-capsule.

6. Complexe incorporant des micro-capsules selon l'une quelconque des revendications 1 à 5, caractérisé en ce qu'il comprend un substrat en forme de feuille, et un revêtement comprenant, sous forme liée au substrat, lesdites micro-capsules.

7. Complexe incorporant des micro-capsules selon l'une quelconque des revendications 1 à 6, caractérisé en ce que les micro-capsules sont en solution ou en suspension dans un liquide, ou fluide préservant l'intégralité de l'enveloppe desdites micro-capsules.

8. Procédé d'encapsulation et libération d'une matière active, consistant à incorporer ladite matière active dans des micro-capsules, obtenues par polymérisation en phase liquide hétérogène d'au moins un monomère, caractérisé en ce qu'au moins l'un des composants des micro-capsules étant susceptible d'absorber et de s'échauffer sous l'effet d'un rayonnement micro-onde, on rompt lesdites micro-capsules par absorption de ces dernières, d'un rayonnement micro-onde, moyennant quoi la matière active est libérée vers l'extérieur.

## Patentansprüche

1. Mikro-Kapsel mit einer undurchlässigen Hülle, die fähig ist, unter der Wirkung einer äußeren Kraft aufzubrechen, mit einer aktiven Substanz und ggf. einem in der Hülle enthaltenen inneren Milieu, dadurch gekennzeichnet, daß wenigstens einer der Bestandteile der Mikro-Kapseln, und zwar die Hülle, die aktive Substanz und ggf. das innere Milieu dazu fähig ist, elektromagnetische Höchstfrequenzstrahlung, Mikrowellenstrahlung genannt, zu absorbieren und sich unter deren Einfluß zu erhitzen, und durch sein Erhitzen zum Zerreißen derselben Mikro-Kapseln unter Freisetzen der aktiven Substanz nach außen zu führen.

2. Mikro-Kapsel nach Anspruch 1, dadurch gekennzeichnet, daß der die Mikrowellenstrahlung absorbierende Bestandteil die Hülle der Mikro-Kapsel ist.

3. Mikro-Kapsel nach Anspruch 2, dadurch gekennzeichnet, daß die Hülle aus einem chemisch-polaren Material erzeugt ist.

4. Mikro-Kapsel nach Anspruch 1, dadurch gekennzeichnet, daß der die Mikrowellenstrahlung absorbierende Bestandteil die aktive Substanz ist.

5. Mikro-Kapsel nach Anspruch 1 mit einem flüssigen inneren Milieu, in dem die aktive Substanz gelöst oder in Suspension ist, dadurch gekennzeichnet, daß der die Mikrowellenstrahlung absorbierende Bestandteil das innere Milieu der Mikro-Kapsel ist.

6. Komplex, welcher Mikro-Kapseln nach einem der Ansprüche 1 bis 5 enthält, dadurch gekennzeichnet, daß er ein blattförmiges Substrat und einen die Mikro-Kapseln aufweisenden Überzug in mit dem Substrat verbundener Form umfaßt.

7. Komplex, welcher Mikro-Kapseln nach einem der Ansprüche 1 bis 6 enthält, dadurch gekennzeichnet, daß die Mikro-Kapseln in Lösung oder in Suspension mit einer Flüssigkeit oder einem Fluid sind, welches die Unversehrtheit der Hülle der Mikro-Kapseln bewahrt.

8. Verfahren zum Einschließen und Freisetzen einer aktiven Substanz, bestehend aus dem Einbringen der aktiven Substanz in Mikro-Kapseln, die durch Polymerisation in heterogener flüssiger Phase von wenigstens einem Monomer erhalten sind, dadurch gekennzeichnet, daß wenigstens einer der Bestandteile der Mikro-Kapseln fähig ist, Mikrowellenstrahlung zu absorbieren und sich unter deren Wirkung zu erhitzen, und man die Mikro-Kapseln durch Absorption der letzteren, einer Mikrowellenstrahlung, zerbricht, wodurch die aktive Substanz in die Umgebung freigesetzt wird.

## Claims

1. Microcapsule comprising a leakproof shell capable of breaking under the action of an external agent, an active substance, and optionally an internal medium, which are contained by the said shell, characterised in that at least one of the components of the microcapsule, namely the shell, the active substance and optionally the internal medium, is capable of absorbing and of self-heating under the effect of an ultrahigh-frequency electromagnetic radiation, known as microwave radiation, and of resulting, through its heating, in the rupture of the same microcapsule, with release of the active substance outwards.

2. Microcapsule according to Claim 1, characterised in that the component absorbing the microwave radiation is the shell of the said microcapsule.

3. Microcapsule according to Claim 2, characterised in that the shell is made of a polar chemical material.

4. Microcapsule according to Claim 1, characterised in that the component absorbing the microwave radiation is the active substance.

5. Microcapsule according to Claim 1, containing a liquid internal medium in which the active substance is dissolved or in suspension, characterised in that the component absorbing the microwave radiation is the internal medium of the microcapsule.

6. Composite incorporating microcapsules according to any one of Claims 1 to 5, characterised in that it comprises a substrate in sheet form and a coating comprising the said microcapsules in a form bonded to the substrate.

7. Composite incorporating microcapsules according to any one of Claims 1 to 6, characterised in that the microcapsules are in solution or in suspension in a liquid or fluid preserving the integrity of the shell of the said microcapsules.

8. Process for encapsulating and releasing an active substance, consisting in incorporating the said active substance in microcapsules obtained by polymerisation of at least one monomer in a heterogeneous liquid phase, characterised in that, at least one of the components of the microcapsules being capable of absorbing and of self-heating under the effect of a microwave radiation, the said microcapsules are broken by absorption of a microwave radiation by the latter, whereby the active substance is released outwards.